# EUROPEAN PATENT APPLICATION

(11) **EP 2 829 301 A1**
(43) Date of publication of application: **28.01.2015**
(21) Application number: 13178073.6
(22) Date of filing: 25.07.2013
(51) Int. Cl.: A61N 5/10, A61M 37/00, A61B 6/04, A61B 19/00, A61B 17/00

(54) **Medical device for radiotherapy treatment**

(71) Applicant: Escarguel, Bruno, 83110 Sanary (FR)
(72) Inventor: Escarguel, Bruno, 83110 Sanary (FR)
(74) Representative: Novagraaf Technologies

(57) **Abstract**

The invention relies on a medical device for an intracorporeal location, comprising a deformable armature, at least partially made of a shape memory material and having an Ea end and an Eb end, said Ea end being larger than said Eb end.

The invention also relies on a device for the introduction, by an endoscopic or percutaneous way, of the medical device, and on a method for accurately targeting a target area during radiation treatment through image guidance comprising determining the location of the medical device in real time using at least one method of targeting selected from the group consisting of lasers, visual, infrared, MRI/MRS, RF and radiation; and modifying the radiation treatment beam path to adaptively compensate for a change in position of the target area.

## Description

### Technical field

The present invention refers to a medical device for an intracorporeal location, a device for the introduction, by an endoscopic or percutaneous way, of said medical device, and to a method for accurately targeting a target area during radiation treatment through image guidance.

Therefore, the present invention has utility in the medical and surgical field.

In the description below, the references into brackets ([ ]) refer to the listing of references situated at the end of the text.

### Background of the Invention

State of the art cancer radiation therapy is increasingly based on the pin point application of high energy radiation which is highly tailored to the shape and position of the cancerous tumor. Modern techniques such as IMRT (intensity-modulated radiation therapy) use a pencil sized beam whose cross-section is shaped to match the tumor. This allows the physician to spare the surrounding healthy tissue while increasing the treatment dose to the cancerous target. As the size of the treatment beam decreases, the accurate location of the beam becomes much more critical. If a highly tailored beam is off target by a few millimeters, it may miss the tumor entirely.

Because of these new techniques, it becomes increasingly desirable to know the position and shape of the tumor accurately with the patient in the exact position that he will be at the time of treatment. In addition, it is critical to be able to place the patient in the same position for multiple fractions of treatment and to be able to confirm that accurate positioning has been accomplished. For this reason, manufacturers of radiation therapy machines are increasingly combining their machines with built in diagnostic imaging capability.

Advances such as On Board Imaging (OBI) and Cone Beam CT allow the verification of patient positioning in real time and the ability to confirm through x-ray that the patient is in the same position as during simulation.

It is also now common for medical clinicians to obtain high contrast and high spatial resolution CT (Computed tomography) and magnetic resonance (MR) data sets. These data sets can be obtained with high spatial resolution between contiguous image slices. These data sets allow for the reconstruction of a precise 3-dimensional (3D) model that accurately describes both the patient's external and internal anatomy. The patient specific models can be manipulated to provide reconstructed views along orthogonal or oblique planes through the patient's anatomy. These computed views allow for clinicians to carry out virtual treatments (or virtual planning) to better optimize therapy for a patient.

Virtual planning is used in several different types of therapy. Radiosurgery, stereotactic radiation therapy, and routine radiotherapy are all therapies that rely upon virtual planning to position radiation beams. Image guided surgery relies upon virtual planning to allow the surgeon to design and evaluate various surgical approaches and to target specific tissues. The virtual planning process places a unique requirement on the basic 3D image data set, that being the ability to track the patient, at the time of therapy, relative to the therapeutic tool. For radiosurgery, stereotactic radiation therapy and radiation therapy, the therapeutic tool is the radiation-generating device, most commonly, a medical linear accelerator. In the case of image guided surgery, the therapeutic tool can be one of a number of devices that the surgeon may use. For example, scalpels, biopsy needles, and operating microscopes are a few of the most commonly guided surgical tools.

In order to provide the required patient-tool tracking both the tool's position and the patient's position must be known. The most common method of tracking the patient is to place identifiable reference markers, called fiducial markers, fixed relative to the portion of the patient where treatment is desired. These markers are incorporated into the 3D image data set. They are also available for identification, again on the surface of the patient (fixed relative to the portion of the patient), at the time of the therapeutic procedure. The markers on the patient are registered against their images in the 3D data set. This registration allows the computation of a rigid relationship between the virtual 3D patient and the real patient.

Once this registration has been carried out, any movement of the patient can be tracked.

This ability to potentially employ positional comparison through imaging on the treatment machine provides the opportunity to develop technologies to discretely locate the patient immobilization devices on the treatment machine and to compare the position to that of the simulation. The imaging technology in treatment and simulation do not have to be the same, and multiple imaging technologies may be employed at each stage, be it x-ray based, MRI (Magnetic resonance imaging) or other modalities. New localization techniques such as the radiofrequency technology developed by Calypso Medical Systems of Seattle present new opportunities to identify and confirm the accuracy of repeated patient positioning. Corrections may be made to the position and orientation of the patient support devices so that accurate targeting of the tumor can be achieved. In addition, the ability to align the couchtop and devices through imaging techniques on the treatment machine allow the process to be proceduralized and automated so that less time is required, increasing productivity.

Traditionally, patient treatment plans have been performed on a separate simulation machine which uses diagnostic imaging either through static images, CT (Computed tomography) imaging, MRI, PET (Positron emission tomography), SPECT (Single Photon Emission Computed Tomography) or other techniques. The patient is placed on a table top also referred to as a couch top. Couch tops developed for Radiation Therapy are generally of a different configuration than those made for diagnostic imaging.

Radiosurgery is an effective tool for the treatment of abnormal lesions, such as malignant cancers. Stereotactic radio surgery ("SRS"), combines the principles of stereotaxy (3-D target localization) with multiple cross-fired beams from a high-energy radiation source to precisely irradiate an abnormal lesion within a patient. This technique allows maximally aggressive dosing of the treatment target, while normal surrounding tissue receives lower, non-injurious doses of radiation.

Several SRS systems are available, including cobalt-sourced systems (also known as GAMMA KNIFE®, Elekta Instruments AB, Sweden), a Swedish and linear accelerator ("LINAC") based devices, such as modified linear accelerators and frameless SRS (e.g., CYBERKNIFE®, Accuray, Sunnyvale, Calif.).

GAMMA KNIFE® employs radioactive cobaltbased gamma ray, whereas LINAC-based systems use X-ray beams generated from a linear accelerator. As a result, the LINAC-based devices do not require or generate any radioactive material. One disadvantage associated with GAMMA KNIFE or conventional LINAC radiosurgery is that a metal head frame is required to be attached to the skull of a patient undergoing brain surgery, and is used to precisely target the radiation beam.

The most advanced LINAC-based system is frameless SRS, which incorporates a miniature linear accelerator mounted on a robotic arm to deliver concentrated beams of radiation to the treatment target from multiple positions and angles. Frameless SRS also employs a realtime x-ray-based image-guidance system to establish the position of the treatment target during treatment, and then dynamically brings the radiation beam into alignment with the observed position of the treatment target. Thus, frameless SRS is able to compensate for patient movement without the need for the invasive and uncomfortable head frame to ensure highly accurate delivery of radiation during treatment. As result, the patient's treatment target receives a cumulative dose of radiation high enough to control or kill the target cells while minimizing radiation exposure to surrounding healthy tissue. With sub-millimeter accuracy, frameless SRS can be used to treat tumors, cancers, vascular abnormalities and functional brain disorders. Frameless SRS can achieve surgical-like outcomes without surgery or incisions. Through the combination of a flexible robotic arm, LINAC, and image guidance technology, frameless SRS is able to reach areas of the body that are unreachable by other conventional radiosurgery systems, including the prostate.

When areas of the body outside the brain are targeted by radiosurgery, the technique is sometimes alternatively referred to as SBRT-stereotactic body radiotherapy. The terms "SRS" and "SBRT" have been used interchangeably by different practitioners to describe the same medical procedure.

Radiosurgery differs from conventional radiotherapy in several ways. The efficacy of radiotherapy depends primarily on the greater sensitivity of tumor cells to radiation in comparison to normal tissue. With all forms of standard radiotherapy, the spatial accuracy with which the treatment is focused on the tumor is less critical compared with radiosurgery; because normal tissues are protected by administering the radiation dose over multiple sessions (fractions) that take place daily for a period of a few weeks. This form of radiation is more effectively repaired by normal tissues, whereas radiosurgery is far more likely to ablate all normal tissues in the high dose zone. As such, radiosurgery, by its very definition, requires much greater targeting accuracy. With SRS, normal tissues are protected by both selective targeting of only the abnormal lesion, and by using cross-firing techniques to minimize the exposure of the adjacent anatomy. Since highly destructive doses of radiation are used, any normal structures (such as nerves or sensitive areas of the brain) within the targeted volume are subject to damage as well. Typically, SRS is administered in one to five daily fractions over consecutive days. Nearly all SRS is given on an outpatient basis without the need for anesthesia. Treatment is usually well tolerated, and only very rarely interferes with a patient's quality of life. Accordingly, SRS has been used to treat benign and malignant tumors, vascular malformations, and other disorders with minimal invasiveness.

Radiosurgical treatment generally involve several phases. First, a three-dimensional map of the anatomical structures in the treatment area is constructed using an imaging technique, such as PET scanning, SPECT, perfusion imaging, tumor hypoxia mapping, angiogenesis mapping, blood flow mapping, cell death mapping, CT, or MRI. Generally, imaging methods, such as CT or nuclear spin tomography, are used to determine the outer contours of the region to be irradiated, such as an outer contour in most cases being marked on the tomographic images obtained. Next, a treatment plan is developed to deliver a dose distribution according to the three-dimensional map. Finally, a patient is treated according to the treatment plan with an appropriate radiosurgical technique. When performing fractionated radiotherapy, accuracy in applying the radiation is very important. Some tumors or other conditions require that the radiation be concentrated in relatively small volumes. Misalignment of the radiation beam may cause an insufficient amount of radiation to be applied to the tumor or other target. Further, such misalignment may increase the likelihood and/or degree of damage to healthy tissue adjacent the tumor or other target.

Fractionated radiotherapy may be imprecise if the tumor or other target cannot be localized with a sufficient degree of accuracy. However, this need for proper localization is the same need which one has when performing single dose radiotherapy and this need is addressed by the present inventors' two first listed below incorporated by reference patents. The additional factor in fractionated radiotherapy is the need to easily and accurately repeat a position of the patient. If the position of the patient was accurate relative to the first treatment, and relative to the imaged data set used to design the treatment, the repositioning should normally cause the patient to assume the exact same position (relative to the treatment mechanism) for the second and subsequent treatments. However, if the second or other subsequent treatment is performed with the patient only slightly moved from the first treatment position, this will introduce inaccuracies. The repeat fixation techniques discussed above have the indicated disadvantages.

More generally, the need for repeat fixation of a patient or portion of a patient exists outside of radiotherapy. In the general case, one wishes to perform a first medical procedure on a patient with a precise localization of portions of the patient, and, at some later time, perform a second medical procedure on the patient with a precise localization of portions of the patient. One can repeat laborious and time-consuming localization steps for the second medical procedure, but this increases medical costs and complexity. As used herein, a medical procedure is a procedure for diagnostic and/or remedial purposes.

In some situations, a single medical treatment without a need for repeat fixation is the desired course of treatment. However, a high degree of accuracy in positioning may still be required. The mechanical arrangements and the associated techniques of the present inventors' last mentioned above two above incorporated by reference patents can provide a high degree of accuracy in positioning of the patient relative to the medical apparatus.

In the radiotherapy procedure, once the reference frame has been removed from the patient the relationship between intracranial target points and the reference system is lost. Because the above procedure would require the reference frame to remain fixed to the patient's skull through the entire course of treatment, which may las several weeks, this approach is considered inappropriate for fractionated therapy. Alternatively, each fractional treatment would require a laborious and time-consuming procedure to re-determine patient position for second and subsequent treatments.

Another setting would be where the patient undergoes a medical imaging procedure to be followed by stereotactic or optic guided surgery. In this setting the patient is scanned prior, sometimes a day or two before, the surgical procedure. The registration of the patient's scan data set to their position on the operative setting is carried out through the use of surface fiducials. This usually entails the shaving of the patient head.

There exist several different techniques for non-invasive repeat fixation. These methods can be broken down into three basic categories. These are bite plate systems, contour realignment systems and mask systems. All of these systems have design flaws which can lead to unacceptable, and undetectable, positional errors.

Diagnostic exams may include a set of fiducial markers whoch allow all points within the image to be localized relative to the stereotactic reference frame. All of these fiducial system attach to the stereotaxic frame in a precision and reproducible manner.

The document US5027818 describes for example techniques for providing stereotactic radiosurgery with a high degree of precicion, by allowing the patient to be precisely positioned relative to radiation beams of stereotactic radiosurgery to within 0.2 mm plus or minus 0.1 mm. These techniques work for single fraction therapy, but are not optimal for clinical settings where fractionating the total dose.

In document US 5,954,647 various locators using bite plates, head rings, and/or masks are described. The bite plates are used for repeat fixation medical procedures (i.e., where procedure is performed with locator on patient, locator and any other associated parts are then removed from the patient, and, at a later time, locator is placed back on patient).

Document US20080031414 describes a patient couch top or device for quickly and accurately positioning a patient during simulation and treatment by placing a series of small fiducial markers in discrete locations on the couch top or device. The use of the fiducial markers allows for the correction for misalignment and deformation of patient positioning equipment which occurs due in part to a patient's size and weight. This document also describes a method for positioning a patient and correcting for deformation of the couch top or device.

However, a high degree of accuracy in positioning may still be required. Accordingly, a need exists to develop markers for deployment and implantation in soft tissues, such as bronchi or bronchioli.

### Description of the Invention

The present invention overcomes the above limitations of the prior art and provides a method to quickly and accurately locate the tumor during simulation and treatment and correct for misalignment and deformation of tumor positioning equipment which occurs due to the patient or organ movements.

The present invention provides a medical device for an intracorporeal location, comprising a deformable armature, at least partially made of a shape memory material and having an Ea end and an Eb end, said Ea end being larger than said Eb end.

"Memory shape material" refers, according to the invention, to any materials that can return to some previously defined shape or size when subjected to the appropriate thermal procedure. Advantageously, the memory shape material may be deformed at ambient temperature, and may recover its original shape when exposed to intra-body temperature. Advantageously, the medical device may be deformed at ambient temperature to adapt its shape to penetrate and circulate into the body and to attain the target tumor or the organ. For example, the deformed shape may be adapted to a circulation of the medical device into the blood vessels or into the lumen of a body cavity, or into a guide suited for the intracorporeal introduction, for example a catheter. For example, the deformed shape may be for example a filament, a cylinder, a cone, a trapeze or a triangle. In this case, the deformed shape may have a length comprised between 2 and 50 mm, for example between 5 and 40 mm, or for example between 10 and 30 mm, and may have a circular, conical or oval basis having a surface comprised between 0.1 cm2 and 30 cm2, for example between 0.5 and 20 cm2, or for example between 1 and 10 cm2. The medical device returns to its original shape with an Ea end and an Eb end, the Ea end being larger than said Eb end, when it is implanted into the body at the target site. Advantageously, stent deployment immediately makes keeping up grace to its structural form.

Advantageously, memory shape material is made of an alloy, for example nickel-titanium alloy, iron-platinum alloy, copper alloy systems, gold-cadmium alloy, nickel-aluminum alloy.

The medical device may comprise a predetermined breaking point. The predetermined breaking point may be on the armature of the medical device. Advantageously, the predetermined breaking point make the implantation into the body or the withdrawal of the medical device outside of the body easier.

The medical device may comprise a mean of fastening and or/opening providing the predetermined breaking point. The mean of fastening and/or opening may be any system allowing sides of the armature of the medical device to stretch out or to come closer. For example, the mean of fastening and/or opening may be made of a metal ball joint with siliconne precut. Advantageously, the fastening and or/opening make the implantation into the body or the withdrawal of the medical device outside of the body easier.

Advantageously, at least one x-ray visible marker may be fixed at the deformable armature. The x-ray visible marker may be selected from the group including, but not limited to, gold, silver, platinum, tantalum, tungsten, niobium, palladium, iridium. For example, one, or two, or three, or at least four x-ray visible marker(s) may be fixed at the deformable armature.

The medical device may include a deformable material forming a surface of the medical device.

"Deformable material" refers, according to the invention, to any material that may change its shape when the memory shape material change its shape. In other words, the deformable material may stretch out or retract depending on the shape of the memory shape material. The deformable material may cover all or part of the surface of the medical device. The deformable material may be micro-perforated. Alternatively, the deformable material may be porous. Advantageously, the microperforations or porosities comprise one or more active substance chosen among the group comprising a therapeutic substance or a diagnostic substance. Advantageously, the active substance is released at the site of a tumor or near the site of a tumor into the body. Whatever the shape of the medical device according to the invention, the deformable material may extend membranous between spaces of the armature to form a concave sail. For example, the membranous material may be silicone.

The deformable material may comprise a fragility zone. The fragility zone may be any area of the deformable material that does not have the same robustness than the rest of the deformable material. In other words, the fragility zone may be any area on the deformable material that is more deformable than the rest of the deformable material. For example, the fragility zone may be an area on the deformable material that does not have the same density than the rest of the deformable material, for example a pre-cut zone or a pre-perforated zone. Advantageously, the fragility zone may join the predetermined breaking point, in order to make the implantation into the body or the withdrawal of the medical device outside of the body easier. The fragility point may be a pre-cut zone at the predetermined breaking point.

The medical device may have a concave curvature. In other words, the armature of the medical device may have a concave curvature when it is stretched out. In this case, the deformable surface also has a concave curvature. Advantageously, the concave curvature allows the medical device to be adapted to the shape of organs of tumors, for example to follow all or part of the tumor or organ and be spatialy visible in a lateral view.

The medical device may have an essentially triangular or trapezoidal shape. For example, the medical device may have essentially the shape of a sail. Advantageously, the medical device may have an essentially triangular or trapezoidal shape, with the Eb end forming the top side of said essentially triangular or trapezoidal shape, wherein the Ea end forms the basis of said essentially triangular or trapezoidal shape located opposite the Eb end and having sides extending between the Eb end and the corners of the Ea end, wherein the essentially triangular or trapezoidal area spanned between the sides and the base is concavely curved.

When the medical device have an essentially triangular or trapezoidal shape, the sides of the essentially triangular or trapezoidal shape extend between said Eb end and the corners of said Ea end. Advantageously, the sides may be curved outwards. Advantageously, the essentially triangular or trapezoidal shaped armature includes a middle rip extending from said Ea end to said Eb end and multiple cross elements extending between the middle rip and said sides, wherein the sides are formed by side braces. Advantageously, the Ea end may be formed by at least two base struts connected at the corners of the Ea and run with a spacing to each other in a middle section of the Ea end to form in combination wih the Eb end a 3-dimensional structure of the armature.

Medical device according to any one of the preceding claims, having a height in the range of 2 and 50 mm. The Ea end of the medical device in its original shape may have a length comprised between 2 and 50 mm.

The sides extending between the Eb end and the corners of the Ea end may have a length comprised between 2 and 50 mm, for example between 5 and 40 mm, or for example between 10 and 30 mm, when the medical device is in its original shape.

The medical device may comprise a system of radio guidance or a transponder. Advantageously, radio guidance or transponder helps better deliver radiation therapy to cancerous tumors, in particular since tumors can move between treatments due to differences in organ filling or movements while breathing. Transponder or radio guidance, as image-guided radiation therapy (IGRT), may involve conformal radiation treatment guided by specialized imaging tests, such as CT scans, ultrasound or X-rays. Transponder or radio-guidance as IGRT may be used during the process of two and three-dimensional imaging, during a course of radiation treatment, to direct radiation therapy utilizing the imaging coordinates of the actual radiation treatment plan. Advantageously, the patient is localized in the treatment room in the same position as planned from the reference imaging dataset. An example of Three-dimensional (3D) IGRT would include localization of a cone-beam computed tomography (CBCT) dataset with the planning computed tomography (CT) dataset from planning. Similarly Two-dimensional (2D) IGRT would include matching planar kilovoltage (kV) radiographs fluoroscopy or megavoltage (MV) images with digital reconstructed radiographs (DRRs) from the planning CT. Advantageously, combination of the radio-guidance and the transponder with x-ray visible marker allows to know exactly where the prostate is at any given time and increase the success of treatment while minimizing exposure to nearby tissues.

Another object of the invention is a device for the introduction, by an endoscopic or percutaneous way, of the medical device as defined above, comprising a guide suited for the intracorporeal introduction, and an extraction holder of the medical device. Advantageously, the medical device circulate inside the device for the introduction towards the target site in the body in its deformed shape, and may recover its original shape once its has attained the target site in the body.The guide may be a catheter for a percutaneous, endoscopic or laparoscopic introduction.

Another object of the invention is a method for accurately targeting a target area during radiation treatment through image guidance comprising determining the location of a medical device as defined above in real time using at least one method of targeting selected from the group consisting of lasers, visual, infrared, MRI/MRS, RF and radiation; and modifying the radiation treatment beam path to adaptively compensate for a change in position of the target area.

For example, a medical method of treatment of a patient in need thereof may comprise the steps, not necessarily in order, of :
- positioning a patient for a first medical procedure;
- positioning the medical device of the invention a first time to get precise positioning information relative to at least part of the patient;
- registering the exact position of the medical device relative to a portion of the patient in a registry;
- performing a first medical procedure on the patient;
- after the first medical procedure, removing the medical device from the patient;
- at a later time after the removing of the medical device, reattaching the medical device to the patient, the medical device again being in registry with the portion of the patient and having an identical relative to the portion of the patient as when the medical device was previously deployed;
- after the re-deployment step, using the medical device a second time to get precise positioning information relative to the at least part of the patient; and
- after the re-deployment step, performing a second medical procedure on the patient.

The invention is further illustrated by the following examples with regard to the annexed drawings not be construed as limiting.

### Description of the Figures

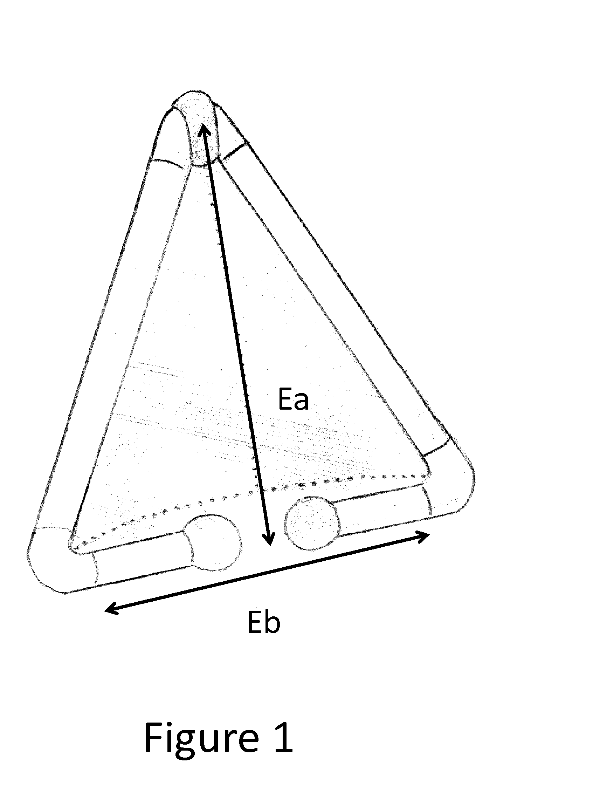

**Figure 1****:** shows a front view of the sail stent, with the Ea end and the Eb end.

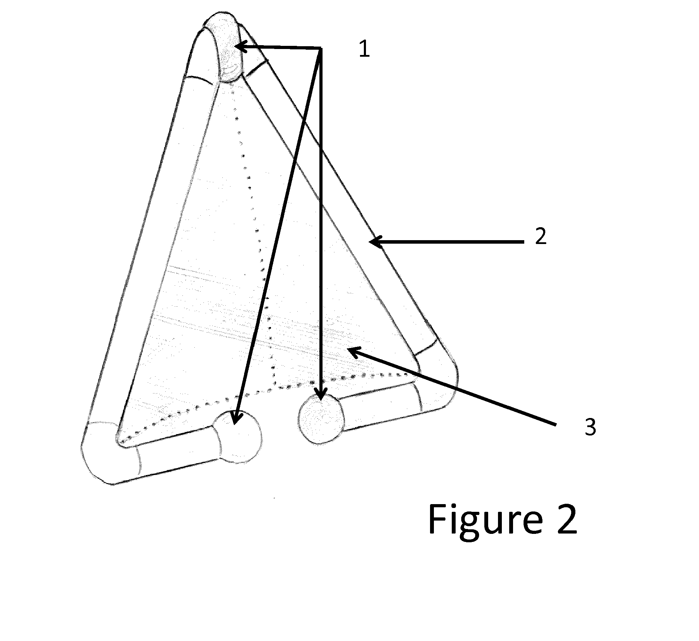

**Figure 2****:** shows a front view of the sail stent, with X-ray visible markers (1), an memory shape armature (2) and a silicone membranous material (3) for the elution of active products or communicating systems. The membranous material is pre-cut for its extraction ("Real Eaze System").

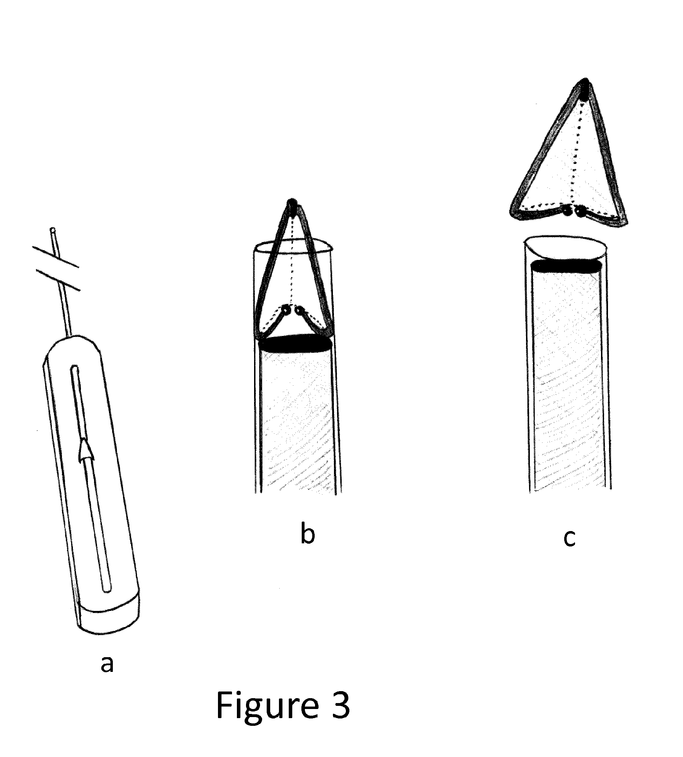

**Figure 3****:** shows the delivery system of the stent. (a): catheter with handle for dropping of the stent. (b) front view of the catheter containing the stent and of the sail stent. (c): front view of the catheter after deployment of the stent.

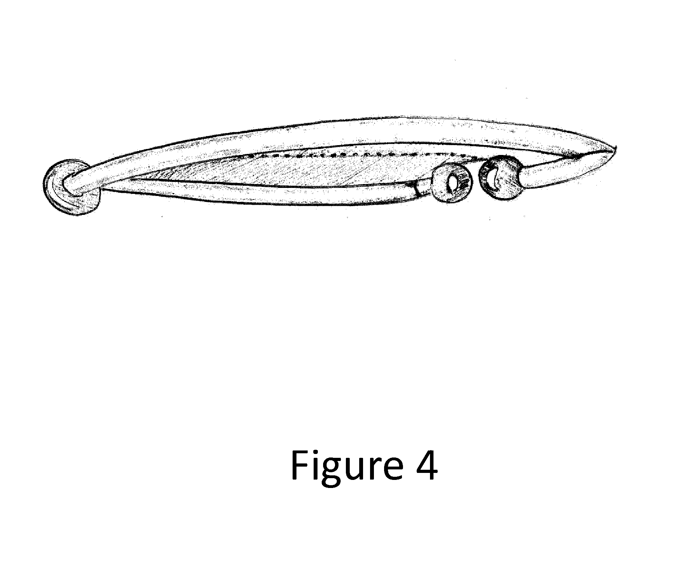

**Figure 4****:** shows a view from the side of the sail stent.

### Examples

### Example of a clinical case

Patient of 58 years, smoking, with a chest opacity of the right upper lobe, is admitted.

PET is positive in the ipsilateral hilar and mediastinal. The rest of the staging does not find any extra thoracic lesion.

The diagnosis of squamous cell carcinoma is confirmed by flexible bronchoscopy using a technique of distal guide ultrasound mini probe. Mediastinal staging is made by an Endobronchial Ultrasound. Therapeutic decision making in multidisciplinary staff is a radio concomitant chemotherapy for stage IIIA disease classified.

Before the medical treatment, system sail stent is positioned within the tumor endoscopically.

The procedure is performed under general anesthesia. The bronchoscope goes through natural means. The same way as for the diagnosis, the lesion is found through ultrasound guidance system. The catheter is left in place within the tumor. The delivery system is inserted into the guide catheter. The sail stent is deployed within the tumor. Confirmation of correct positioning of the stent method is verified by fluoroscopy or ultrasound. The bronchoscope is removed. Procedure time did not exceed 20 minutes. The patient is awake and can enjoy a guided radiotherapy more precisely by the Sail Stent. After the treatment séquence, the stent is left in place or removed endoscopically.

The outlook is based on the possibility of increasing the intensity of radiation complications limiting the healthy tissue. In addition, the use of the stent chemotoxic by elution improve the performance of radiation.

### List of references

- US5027818.
- US 5,954,647.
- US20080031414.

## Claims

1. Medical device for an intracorporeal location, comprising a deformable armature, at least partially made of a shape memory material and having an Ea end and an Eb end, said Ea end being larger than said Eb end.

2. Medical device according to claim 1, wherein at least one x-ray visible marker is fixed a the deformable armature and is selected from the group consisting of gold, silver, platinum, tantalum, tungsten, niobium, palladium, iridium.

3. Medical device according to claim 1 or 2, including a deformable material forming a surface of the medical device.

4. Medical device according to anyone of claims 1 to 3, having a concave curvature.

5. Medical device according to anyone of claim 1 to 4, having an essentially triangular or trapezoidal shape, with the Eb end forming the top side of said essentially triangular or trapezoidal shape, wherein the Ea end forms the basis of said essentially triangular or trapezoidal shape located opposite the Eb end and having sides extending between the Eb end and the corners of the Ea end, wherein the essentially triangular or trapezoidal area spanned between the sides and the base is concavely curved.

6. Medical device according to anyone of the preceding claims comprising a predetermined breaking point.

7. Medical device according to claim 6, comprising a mean of fastening and or/opening providing said predetermined breaking point.

8. Medical device according to claim 7, in which said mean of fastening and/or opening is constituted of a metal ball joint.

9. Medical device according to any one of claims 3 to 8, in which said deformable material is micro-perforated.

10. Medical device according to any one of claims 3 to 9, in which said deformable material comprises a fragility zone.

11. Medical device according to any one of the preceding claims, in which said deformable material comprises an active substance chosen among the group comprising a therapeutic substance or a diagnostic substance.

12. Medical device according to any one of the preceding claims, comprising a system of radio guidance or a transponder.

13. Medical device according to any one of the preceding claims, having a height in the range of 2 to 50 mm.

14. A device for the introduction, by an endoscopic or percutaneous way, of a medical device as defined in any one of claims 1 to 13, comprising a guide suited for the intracorporeal introduction, and an extraction holder of said medical device as defined in any one of claims 1 to 13.

15. A method for accurately targeting a target area during radiation treatment through image guidance comprising determining the location of a medical device as defined in any one of claims 1 to 13 in real time using at least one method of targeting selected from the group consisting of lasers, visual, infrared, MRI/MRS, RF and radiation; and modifying the radiation treatment beam path to adaptively compensate for a change in position of the target area.
